# EUROPEAN PATENT APPLICATION

(11) **EP 4 806 441 A1**
(43) Date of publication of application: **16.09.2026**
(21) Application number: 24889219.2
(22) Date of filing: 11.11.2024
(51) Int. Cl.: A61K 31/722, A61K 35/12, A61P 17/10, A61K 8/73, A61K 8/98, A61Q 7/00

(54) **COMPOSITION COMPRISING CHITOSAN DERIVATIVE AND DECELLULARIZED EXTRACELLULAR MATRIX HYDROLYSATE AND USE THEREOF**

(30) Priority: 10.11.2023 KR 20230155653
(71) Applicant: Medifab Co., Ltd., Seoul 08594 (KR)
(72) Inventor: KIM, Su Hee, Seoul 08594 (KR); CHA, Mi Sun, Seoul 08594 (KR); KANG, Jae Jun, Seoul 08594 (KR)
(74) Representative: Morabito, Sara
(86) International application number: PCT/KR2024/017783
(87) International publication number: WO 2025/101033

(57) **Abstract**

The present application relates to a composition including a chitosan derivative and a decellularized extracellular matrix hydrolysate, and a use thereof. The present application provides a composition including a chitosan derivative, in which a degree of deacetylation, a degree of substitution of an alkylcarboxyl group, and a degree of free amine groups are respectively controlled, and a decellularized extracellular matrix hydrolysate; a composition for improving hair or scalp conditions including the same; and a use of the composition for improving hair or scalp conditions.

## Description

### Technical Field

The present application relates to a composition including a chitosan derivative and a decellularized extracellular matrix hydrolysate, and a use thereof. The present application claims Korean Patent Application No. 10-2023-0155653 filed on November 10, 2023 as a priority, and the disclosure of the patent application is incorporated herein by reference.

### Background Art

Hair loss refers to the phenomenon of the loss of hair from scalp tissue along with itching as the capillaries of the scalp contract due to various factors such as genetic, environmental, and mental factors, which is one of the hidden concerns troubling modern people. The causes of such hair loss include excessive mental stress, intellectual labor, and instant food-based diet, which leads to inadequate blood circulation in the scalp, malnutrition of hair root cells, and deterioration of the function of the endocrine system. As a result, the number of hair root cells growing significantly decreases compared to the number of dying hair root cells, resulting in premature hair loss even during the growth period. Accordingly, there is a need to develop a technology for improving the hair loss phenomenon.

Meanwhile, an extracellular matrix (ECM) is a non-cellular structure of a tissue or organ, which is composed of various polymers secreted from cells. The extracellular matrix may be used as a natural substance that may be used structurally intact and suitable for biological purposes. Various bioactive substances, for example, collagen, glycosaminoglycan, various cytokines, and the like are included in the extracellular matrix, and thus, tissue regeneration may be promoted and inflammation may be alleviated. The extracellular matrix may be used alone or in combination with other materials for tissue regeneration. However, there is a need for improvement in terms of complexation with other materials, formulation, biocompatibility, and maintenance of efficacy.

### Disclosure of Invention

### Technical Problem

An aspect is to provide a pharmaceutical composition for preventing or treating hair loss, the pharmaceutical composition including: a chitosan derivative, in which the degree of deacetylation, the degree of substitution of an alkyl carboxyl group, and the degree of a free amine group are each controlled; and a hydrolysate of a decellularized extracellular matrix.

Another aspect is to provide a composition for improving hair or scalp conditions, the composition including a chitosan derivative, in which the degree of deacetylation, the degree of substitution of an alkyl carboxyl group, and the degree of a free amine group are each controlled; and a hydrolysate of a decellularized extracellular matrix. Other objects and advantages of the present disclosure will become more apparent from the following detailed description together with the appended claims and drawings. Since the contents not described in the present specification can be sufficiently recognized and inferred by those skilled in the technical field of the present application or a similar technical field, the description thereof will be omitted.

### Solution to Problem

Each description and embodiment disclosed in this application may also apply to other descriptions and embodiments. That is, all combinations of various elements disclosed in the present application fall within the scope of the present application. In addition, it should not be considered that the scope is limited by the detailed description described below.

An aspect provides a pharmaceutical composition for preventing or treating hair loss, the pharmaceutical composition including: a chitosan derivative satisfying the following conditions; and a hydrolysate of a decellularized extracellular matrix:
(a) a degree of deacetylation is 70% or more;
(b) 50% or more of the hydroxyl groups and amine groups in the chitosan derivative are substituted with an O-alkylcarboxyl group or an N-alkylcarboxyl group; and
(c) 22% or more of the total amine groups in the chitosan derivative are free amine groups (-NH₂).

The term "chitosan" as used herein may refer to a linear polysaccharide consisting of D-glucosamine and N-acetylglucosamine. The chitosan may include a material obtained by deacetylating chitin using an alkali or the like. More specifically, the chitosan refers to a polymer material having a D-glucosamine content of 70% or more produced by the separation of an acetyl group from N-acetyl-D-glucosamine, and may be represented by Structural Formula 1.

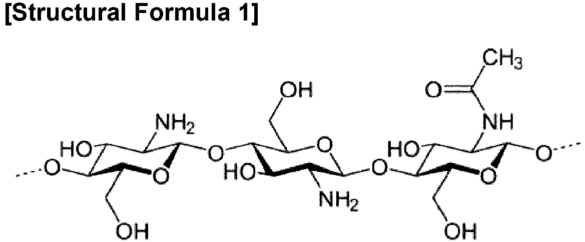

The term "chitosan derivative" as used herein is a material derived from the chitosan described above, and may refer to a structure into which an organic chemical modification including introduction of a specific functional group, oxidation, reduction, substitution of atoms, and the like is introduced. Since conventional chitosan derivatives in the art act as weak polyanionic polyelectrolytes, the amino groups in the chitosan derivatives are not protonated under neutral conditions and most of the carboxyl groups remain insoluble, and thus there is a limitation in using them as biomaterials. Therefore, there is a need to develop a new technique for improving the utility of chitosan derivatives as biomaterials. The chitosan derivative may be, for example, carboxyalkyl chitosan, and specifically, may be composed of a combination of a plurality of units represented by Structural Formula 2 below.

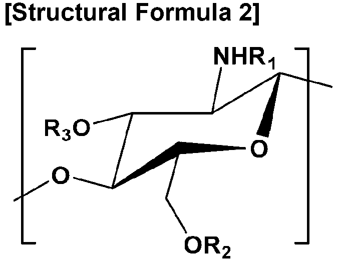

In Structural Formula 2, R_{1 may} be any one of hydrogen, an alkylcarboxyl group, and an acetyl group, and R₂ and R₃ may independently be hydrogen or an alkylcarboxyl group. In this regard, the alkylcarboxyl group may be, for example, a methylcarboxyl group or an ethylcarboxyl group, and specifically, a methylcarboxyl group.

In an embodiment, the chitosan derivative may have an average molecular weight of 50,000 to 3,000,000 Da. The average molecular weight is derived from a plurality of units represented by Structural Formula 1 or Structural Formula 2 described above, or a combination thereof, and may be appropriately changed to a general range known in the art.

In an embodiment, the chitosan derivative may have properties of exhibiting high solubility and stability under neutral conditions. To this end, the chitosan derivative may have a degree of deacetylation of 70% or more, a degree of substitution of 50% or more of an O-alkylcarboxyl group or an N-alkylcarboxyl group, and a degree of the free amine groups of 22% or more. In addition, the pH of the chitosan derivative-containing solution or the composition including the same according to an embodiment may be pH 6.5 to pH 8.0, for example, pH 6.5 to pH 8.0, pH 6.5 to pH 7.5, pH 6.8 to pH 7.5, pH 7.1 to pH 7.5, pH 7.4 to pH 7.5, pH 6.5 to pH 7.2, pH 6.8 to pH 7.2, pH 7.1 to pH 7.2, pH 6.5 to pH 6.9, pH 6.8 to pH 6.9, or pH 6.5 to pH 6.6. The degree of deacetylation of the chitosan derivative described above, the degree of substitution of the O-alkylcarboxyl group or the N-alkylcarboxyl group, and the degree of the free amine groups are parameters that can be adjusted according to known techniques in the art, and may include, for example, a series of processes of reacting chitosan powder having a specific level of deacetylation with an appropriate amount of monochloroacetic acid, but is not limited thereto.

For example, the degree of deacetylation, the degree of substitution of the O-alkylcarboxyl group or the N-alkylcarboxyl group, and the like may be adjusted according to the following method: In order to obtain chitosan derivatives having different levels of deacetylation, 10 g of chitin may be reacted in 100 mL of a 50% NaOH solution at 100 °C for 30 minutes to 150 minutes, for example, 30 minutes to 120 minutes, 30 minutes to 90 minutes, 30 minutes to 60 minutes, 60 minutes to 150 minutes, 60 minutes to 140 minutes, 60 minutes to 130 minutes, 60 minutes to 120 minutes, 60 minutes to 110 minutes, 60 minutes to 100 minutes, 60 minutes to 90 minutes, 60 minutes to 80 minutes, or 60 minutes to 80 minutes. In addition, in order to obtain a chitosan derivative having a different alkyl carboxyl group or degree of the free amine groups, the chitosan derivative powder with a controlled degree of deacetylation as described above may be added in an amount of 5 w/v% to 20% NaOH, and then 1.0 to 1.5% monochloroacetic acid may be added thereto and reacted.

In an embodiment, the chitosan derivative may have a degree of deacetylation of 70% to 95%. The degree of deacetylation indicates a level at which an acetyl group is removed from chitin, which is a precursor of chitosan, and in Structural Formula 2, may indicate a % level of a unit in which R₁ is hydrogen or an alkylcarboxyl group. The degree of deacetylation of the chitosan derivative may be, for example, 70% to 95%, 70% to 90%, 75% to 90%, 80% to 90%, 85% to 90%, 70% to 86%, 75% to 86%, 80% to 86%, 85% to 86%, 70% to 82%, 75% to 82%, 80% to 82%, 70% to 78%, 75% to 78%, or 70% to 74%. In an embodiment, the chitosan derivative may have a degree of substitution of 50% to 90% of O-alkylcarboxyl group or N-alkylcarboxyl group. The degree of substitution of the O-alkylcarboxyl group or the N-alkylcarboxyl group indicates the degree of substitution with the -O-alkylcarboxyl group or the -N-alkylcarboxyl group among the hydroxyl groups and the amine groups in the chitosan derivative, and in Structural Formula 2, R₂ and R₃ may indicate the % level of a substituent which is an alkylcarboxyl group. In this regard, the % level is a value calculated by the number of carboxymethyl groups per 100 anhydroglucosamine units, that is, a concept distinguished from the conventional substitution degree evaluated by the substitution of the carboxyl group. The degree of substitution of the chitosan derivative with the O-alkylcarboxyl group or the N-alkylcarboxyl group may be, for example, 50% to 90%, 55% to 90%, 60% to 90%, 65% to 90%, 70% to 90%, 75% to 90%, 80% to 90%, 85% to 90%, 50% to 80%, 55% to 80%, 60% to 80%, 65% to 80%, 70% to 80%, 75% to 80%, 50% to 70%, 55% to 70%, 60% to 70%, 65% to 70%, 50% to 60%, or 55% to 60%. In this case, when the degree of deacetylation and the degree of substitution of the O-alkylcarboxyl group or the N-alkylcarboxyl group of the chitosan derivative are out of the above ranges, there is a problem in that the stability is rapidly deteriorated under neutral conditions, and thus insoluble particles that are not dissolved in the solution increase.

In an embodiment, the chitosan derivative may have a degree of the free amine groups of 22% to 90%. The degree of the free amine groups represents a degree of the free amine groups (-NH₂) in amine groups in the chitosan derivative, and in Structural Formula 2, it may represent a % level of a substituent where R₁ is hydrogen. The degree of the free amine groups represents a degree of the free amine groups (-NH₂) in amine groups in the chitosan derivative, and in Structural Formula 2, it may represent a % level of a substituent where R₁ is hydrogen. The degree of the free amine groups of the chitosan derivative may be 22% to 90%, 25% to 90%, 30% to 90%, 35% to 90%, 40% to 90%, 45% to 90%, 50% to 90%, 55% to 90%, 60% to 90%, 65% to 90%, 70% to 90%, 75% to 90%, 80% to 90%, 85% to 90%, 22% to 80%, 25% to 80%, 30% to 80%, 35% to 80%, 40% to 80%, 45% to 80%, 50% to 80%, 55% to 80%, 60% to 80%, 65% to 80%, 70% to 80%, 75% to 80%, 22% to 70%, 25% to 70%, 30% to 70%, 35% to 70%, 40% to 70%, 45% to 70%, 50% to 70%, 55% to 70%, 60% to 70%, 65% to 70%, 22% to 60%, 25% to 60%, 30% to 60%, 35% to 60%, 40% to 60%, 45% to 60%, 50% to 60%, or 55% to 60%.

In an embodiment, the chitosan derivative may be present in a state in which a free amine group (-NH₂) in the chitosan derivative is activated, and the activated state may refer to a condition in which the free amine group in the chitosan derivative is present in the form of -NH₃+. For example, the free amine group in the chitosan derivative may be present in an activated state by treating the chitosan derivative with an acidic solution. The treating the acidic solution may be adding chitosan derivative powder to a HCl solution. In addition, the activating the amine group in the chitosan derivative may further include correcting the pH of the solution to a neutral pH after the treating with the acidic solution. Specifically, after treating the chitosan derivative powder with an acidic solution, the method may further include adjusting the pH to 6.5 to 7.0 by treating with a sodium phosphate solution. The state in which the free amine group in the chitosan derivative is activated may refer to a condition in which 20% or more of the free amine groups in the chitosan derivative are present in the form of -NH₃+. By including an activated free amine group, the chitosan derivative exhibits excellent reactive oxygen species (ROS) scavenging activity, inhibits apoptosis, and promotes the regeneration of cells or tissues, thereby providing a superior anti-inflammatory effect.

In an embodiment, the chitosan derivative may have a degree of deacetylation of 70% to 95%, a degree of substitution of an O-alkylcarboxyl group or an N-alkylcarboxyl group of 50% to 90%, and a degree of the free amine groups of 22% to 90%, and the chitosan derivative may have, for example, a degree of deacetylation of 70% to 95%, a degree of substitution of an O-alkylcarboxyl group or an N-alkylcarboxyl group of 50% to 70%, and a degree of the free amine groups of 24% to 90%.

According to an embodiment, it was confirmed that the characteristics of the composition may be adjusted according to the structure of the chitosan derivative. Specifically, the degree of deacetylation of the chitosan derivative, the degree of the terminal carboxyl group (i.e., the degree of substitution of the O-alkylcarboxyl group or the N-alkylcarboxyl group), and the degree of the free amine groups may affect the solubility, stability, formulation and complexation with other components or materials of the chitosan derivative under neutral conditions. Therefore, in the present embodiment, by controlling the degree of deacetylation of a chitosan derivative, the substitution degree of terminal carboxyl groups, and the degree of free amine groups, a composition was developed that exhibits excellent solubility and stability under neutral conditions, can be formulated as a solution, and is readily combinable with other materials. It was thereby confirmed that the composition achieves hair loss prevention or treatment effects and hair or scalp condition improvement effects, and the present disclosure was completed on the basis of these findings.

The chitosan derivative may be contained at a concentration of 0.05 w/v% to 5 w/v% based on the total volume of the chitosan derivative solution, and the content of the chitosan derivative may be 0.05 w/v% to 5 w/v%, 0.1 w/v% to 5 w/v%, 0.15 w/v% to 5 w/v%, 0.2 w/v% to 5 w/v%, 0.05 w/v% to 4 w/v%, 0.1 w/v% to 4 w/v%, 0.15 w/v% to 4 w/v%, 0.2 w/v% to 4 w/v%, 0.05 w/v% to 3 w/v%, 0.1 w/v% to 3 w/v%, 0.15 w/v% to 3 w/v%, 0.2 w/v% to 3 w/v%, 0.05 w/v% to 2 w/v%, 0.1 w/v% to 2 w/v%, 0.15 w/v% to 2 w/v%, 0.2 w/v% to 2 w/v%, 0.05 w/v% to 2 w/v%, 0.1 w/v% to 2 w/v%, 0.15 w/v% to 2 w/v%, 0.2 w/v% to 2 w/v%, 1 w/v% to 5 w/v%, 1.5 w/v% to 5 w/v%, 2 w/v% to 5 w/v%, 2.5 w/v% to 5 w/v%, 3 w/v% to 5 w/v%, 3.5 w/v% to 5 w/v%, 4 w/v% to 5 w/v%, 4.5 w/v% to 5 w/v%, 1 w/v% to 4 w/v%, 1.5 w/v% to 4 w/v%, 2 w/v% to 4 w/v%, 2.5 w/v% to 4 w/v%, 3 w/v% to 4 w/v%, 3.5 w/v% to 4 w/v%, 1 w/v% to 3 w/v%, 1.5 w/v% to 3 w/v%, 2 w/v% to 3 w/v%, or 2.5 w/v% to 3 w/v%, based on the total volume of the chitosan derivative solution. When the content of the chitosan derivative is less than lower limits of these ranges, the anti-inflammatory effect and the resulting hair loss prevention or treatment effect are decreased, and when the content of the chitosan derivative is greater than or equal to the upper limits of these ranges, the insoluble particles not dissolved in the solution increase, and residues remain on the scalp or hair after application and drying of the composition.

The term "decellularized extracellular matrix" as used herein may be used interchangeably with "decellularized matrix", "decellularized tissue", or "decellularized material". The decellularized extracellular matrix refers to a material obtained by performing decellularization on tissues and organs of a human or animals such as pigs or cows to remove cellular components other than the extracellular matrix, for example, nuclei, cell membranes, nucleic acids, and the like. Accordingly, the immunogenicity of the extracellular matrix may be removed or reduced.

In an embodiment, the decellularized extracellular matrix may be prepared by a decellularization method of injecting supercritical carbon dioxide into a reactor including a biological tissue and ethanol such that the pressure in the reactor becomes 70 to 400 bar, wherein the injecting supercritical carbon dioxide into the reactor including the biological tissue and ethanol may be performed at 31 to 60 °C

The biological tissue may be skin tissue, adipose tissue, heart tissue, corneal tissue, or cartilage tissue of a human or an animal such as a pig or a cow, but is not limited thereto. In the injecting supercritical carbon dioxide into the reactor, the putting the biological tissue and ethanol into the reactor and the injecting supercritical carbon dioxide may be performed simultaneously or sequentially, and this may serve to remove components having immunogenicity such as cell components in the biological tissue through high pressure.

The injecting supercritical carbon dioxide into the reactor may be performed at 31 to 60 °C When the temperature in the reactor is outside the temperature range and the temperature in the reactor is low, the decellularization process does not sufficiently occur and thus the immunogenicity of the decellularized matrix may not be sufficiently removed, and when the temperature in the reactor is high, active ingredients such as physiologically active substances in the extracellular matrix may be degraded or denatured in the decellularization process. For example, the treating with the supercritical carbon dioxide may be performed in the reactor at the temperature of 31 to 55 °C, 31 to 50 °C, 31 to 45 °C, 31 to 40 °C, 31 to 39 °C, 31 to 38 °C, 31 to 37 °C, 32 to 40 °C, 33 to 40 °C, 34 to 40 °C, 35 to 40 °C, 32 to 39 °C, 33 to 39 °C, 34 to 39 °C, 35 to 39 °C, 32 to 38 °C, 33 to 38 °C, 34 to 38 °C, 35 to 38 °C, 32 to 37 °C, 33 to 37 °C, 34 to 37 °C, or 35 to 37 °C, but is not limited thereto.

In the injecting supercritical carbon dioxide into the reactor, carbon dioxide may be injected into the reactor such that a pressure in the reactor becomes 70 to 400 bar. When the pressure in the reactor is lower than the lower limit of the pressure range, the decellularization process does not occur sufficiently, and the immunogenicity of the decellularized matrix is not sufficiently removed. For example, supercritical carbon dioxide may be injected such that the pressure in the reactor is 80 to 400 bar, 90 to 400 bar, 100 to 400 bar, 110 to 400 bar, 120 to 400 bar, 130 to 400 bar, 140 to 400 bar, 150 to 400 bar, 150 to 390 bar, 150 to 380 bar, 150 to 370 bar, 150 to 360 bar, 150 to 350 bar, 160 to 350 bar, 170 to 350 bar, 180 to 350 bar, 190 to 350 bar, 200 to 350 bar, 210 to 350 bar, 220 to 350 bar, 230 to 350 bar, 240 to 350 bar, 250 to 350 bar, 260 to 350 bar, 270 to 350 bar, 280 to 350 bar, 290 to 350 bar, or 300 to 350 bar, but is not limited thereto.

The injecting supercritical carbon dioxide into the reactor may be performed for 1 to 24 hours, and for example, may include maintaining the pressure in the reactor for 1 to 24 hours. For example, the injecting may be performed for 1 to 23 hours, 1 to 22 hours, 1 to 21 hours, 1 to 20 hours, 1 to 19 hours, 1 to 18 hours, 1 to 17 hours, 1 to 16 hours, 1 to 15 hours, 1 to 14 hours, 1 to 13 hours, 1 to 12 hours, 2 to 12 hours, 2 to 11 hours, 2 to 10 hours, 2 to 9 hours, 2 to 8 hours, 2 to 7 hours, or 2 to 6 hours, but is not limited thereto. For example, the treating of the supercritical carbon dioxide may include maintaining the pressure in the reactor for 2 to 6 hours.

The decellularization method may further include a pretreatment process before the contacting the biological tissue with ethanol in the reactor. Contaminants, fats, and the like of the biological tissue may be removed through the pretreatment process.

The method of preparing the decellularized extracellular matrix may further include a drying process following the decellularization process, and may subsequently include a pulverization process thereafter. A drying method or a pulverization method commonly used in the art may be used.

The term "hydrolysate" as used herein may include a hydrolyzed solution of decellularized extracellular matrix, a diluted or concentrated solution thereof, a dried product obtained by drying the hydrolyzed solution, a crude or purified product thereof, or a fraction thereof. The hydrolysate of the decellularized extracellular matrix may be hydrolyzed under basic conditions. In an embodiment, the decellularized extracellular matrix hydrolysate may be obtained by treating the decellularized extracellular matrix powder with a basic solution. The basic solution may be a basic aqueous solution. The pH of the basic aqueous solution may be pH 9.5 to 14. For example, the pH of the basic aqueous solution may be pH 9.5 to 13.5, pH 9.5 to 13.0, pH 9.5 to 12.5, pH 10.0 to 13.5, pH 10.0 to 13.0, pH 10.0 to 12.5, pH 10.5 to 13.5, pH 10.5 to 13.0, pH 10.5 to 12.5, pH 11.0 to 13.5, pH 11.0 to 13.0, pH 11.0 to 12.5, pH 11.5 to 13.5, pH 11.5 to 13.0, pH 11.5 to 12.5, pH 12.0 to 13.5, pH 12.0 to 13.0, or pH 12.0 to 12.5. According to an embodiment, it was confirmed that, by including a decellularized extracellular matrix hydrolysate hydrolyzed under basic conditions, the composition may include hair or scalp growth-related factors at a high content, and exhibits an excellent inflammation-alleviatingg effect and an absorption-enhancing effect into the dermal layer. In addition, the effects of the composition on hair root cell proliferative ability and on an increase in the expression level of VEGF in hair root cells were confirmed. As a result, it was confirmed that the composition according to an embodiment may be used for preventing or treating hair loss or improving hair or scalp conditions.

The hydrolysate of the decellularized extracellular matrix may be obtained by treating a solution containing the decellularized extracellular matrix with supercritical carbon dioxide. The treating the supercritical carbon dioxide may be performed at 60 to 150 °C. The treatment with supercritical carbon dioxide is performed in a reactor, and carbon dioxide may be injected such that the pressure in the reactor becomes 70 to 400 bar. According to the method of treatment with supercritical carbon dioxide, the decellularized extracellular matrix may have effects of enhancing biocompatibility, enhancing transdermal absorption, maintaining a physiologically active substance, or facilitating complexation with other materials or components.

The term "prevention" as used herein refers to any action that suppresses or delays the onset of a disease by administration of the composition.

The term "treatment" as used herein refers to any form of treatment that provides an effect, including improvement of a state (e.g., one or more symptoms) of a subject suffering from a disease or at risk of developing a disease, delay of disease progression, delay of occurrence of symptoms, or slowing of symptom progression, to the subject. Thus, the "treatment" and "prevention" are not intended to imply healing or complete elimination of symptoms.

The term "subject" refers to a subject requiring treatment of a disease, and more specifically, refers to a mammal such as a human or non-human primate, mouse, dog, cat, horse, and cow.

The term "hair loss", which is a disease to be prevented or treated by the pharmaceutical composition, may refer to a state in which hair is absent in a region where hair should be present, and specifically, it may refer to loss of terminal hair (thick black hair) of the scalp. The cause of hair loss is not limited, but may be caused by various eating habits and environmental effects such as genetic causes, hormone imbalance, mental stress, exposure to air pollution, and ingestion of processed foods. For example, hair loss may include hereditary androgenic hair loss (baldness), alopecia areata, tinea capitis due to fungal infection, telogen effluvium, trichotillomania, hair growth disorders, and the like, and scarring alopecia in which a scar is formed may refer to hair loss due to lupus, dissecting cellulitis, lichen planopilaris, and hair loss due to burns and trauma.

The pharmaceutical composition may include a pharmaceutically effective amount of the chitosan derivative and the decellularized extracellular matrix hydrolysate; and/or a pharmaceutically acceptable carrier, but is not limited thereto.

The term "pharmaceutically effective amount" as used herein refers to an amount sufficient to achieve the hair loss prevention or treatment efficacy of the pharmaceutical composition.

The pharmaceutically acceptable carrier is commonly used in the preparation of formulations, and includes lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, and the like, but is not limited thereto. Suitable pharmaceutically acceptable carriers and agents are described in detail in Remington's Pharmaceutical Sciences (19th ed., 1995).

The pharmaceutical composition may further include a lubricant, a wetting agent, a sweetening agent, a flavoring agent, an emulsifying agent, a suspending agent, a preservative, and the like, in addition to the components as described above, but is not limited thereto.

The pharmaceutical composition may be administered orally or parenterally, preferably parenterally, and in the case of parenteral administration, it may be administered by skin application, muscle injection, intravenous injection, subcutaneous injection, intraperitoneal injection, topical administration, transdermal administration, and the like, but is not limited thereto.

The dosage of the pharmaceutical composition used may be 0.1 to 10 g, for example, 1 to 10 g per day, but is not limited thereto, and may be variously prescribed depending on factors such as formulation method, administration method, patient's age, body weight, sex, pathological condition, diet, administration time, administration route, excretion rate, and response sensitivity.

The pharmaceutical composition may be prepared in a unit-dose form or by being filled into a multi-dose container by formulating with a pharmaceutically acceptable carrier and/or excipient according to a method readily implementable by a person of ordinary skill in the art to which the present disclosure pertains

In an embodiment, the composition may enhance cell viability of dermal papilla cells or increase expression of vascular endothelial growth factor (VEGF) in dermal papilla cells. According to an embodiment, it was confirmed that the composition has the effects of alleviating inflammation, promoting viability or cell activity of dermal papilla cells, promoting expression of hair growth-related factors, and promoting hair growth, and thus the composition may be used as a composition for preventing or treating hair loss.

Another aspect provides a method of preparing a pharmaceutical composition for preventing or treating hair loss, the pharmaceutical composition including a chitosan derivative satisfying the following conditions and a decellularized extracellular matrix hydrolysate:
(a) a degree of deacetylation is 70% or more;
(b) 50% or more of the hydroxyl groups and amine groups in the chitosan derivative are substituted with an O-alkylcarboxyl group or an N-alkylcarboxyl group; and
(c) 22% or more of the total amine groups in the chitosan derivative are free amine groups (-NH₂),

the method including: preparing a chitosan derivative; and
preparing a decellularized extracellular matrix hydrolysate.

In an embodiment, the preparing a hydrolysate of the decellularized extracellular matrix may include decellularizing an extracellular matrix, wherein the decellularizing the extracellular matrix may include a decellularization process of injecting supercritical carbon dioxide into a reactor including a biological tissue and ethanol such that the pressure in the reactor becomes 70 to 400 bar, and the injecting supercritical carbon dioxide into the reactor including the biological tissue and ethanol may be performed at 31 to 60 °C

In an embodiment, the preparing a hydrolysate of the decellularized extracellular matrix may include treating the decellularized extracellular matrix with supercritical carbon dioxide, wherein the treating the supercritical carbon dioxide may be performed at 60 to 150 °C. The treatment with supercritical carbon dioxide is performed in a reactor, and carbon dioxide may be injected such that the pressure in the reactor becomes 70 to 400 bar.

In an embodiment, the preparing the chitosan derivative may include activating a free amine group in the chitosan derivative by treating, with an acidic solution, the chitosan derivative powder having the conditions as described above. The activated state may refer to a condition in which the free amine group in the chitosan derivative exists in the form of -NH₃+. The treating with the acidic solution may be adding a chitosan derivative powder to the HCl solution.

In an embodiment, the preparing the chitosan derivative may further include correcting the pH of the solution to a neutral pH after the treating the chitosan derivative powder with an acidic solution. For example, the method may further include treating the chitosan derivative powder with a sodium phosphate solution to adjust the pH to 6.5 to 7.0 after treating the chitosan derivative powder with an acidic solution and then.

Another aspect provides a method of preventing or treating hair loss, the method including administering a therapeutically effective amount of a pharmaceutical composition for preventing or treating hair loss, which includes a chitosan derivative satisfying the following conditions; and a hydrolysate of a decellularized extracellular matrix, to a subject:
(a) a degree of deacetylation is 70% or more;
(b) 50% or more of the hydroxyl groups and amine groups in the chitosan derivative are substituted with an O-alkylcarboxyl group or an N-alkylcarboxyl group; and
(c) 22% or more of the total amine groups in the chitosan derivative are free amine groups (-NH₂). The foregoing descriptions also apply equally to the method of preventing or treating hair loss.

According to another aspect of the present disclosure, there is provided a composition for improving hair or scalp conditions, the composition including: a chitosan derivative that satisfies the following conditions; and a hydrolysate of a decellularized extracellular matrix:
(a) a degree of deacetylation is 70% or more;
(b) 50% or more of the hydroxyl groups and amine groups in the chitosan derivative are substituted with an O-alkylcarboxyl group or an N-alkylcarboxyl group; and
(c) 22% or more of the total amine groups in the chitosan derivative are free amine groups (-NH₂). The foregoing descriptions also apply equally to the composition for improving hair or scalp conditions.

In an embodiment, the improvement in hair or scalp conditions may correspond to hair growth promotion, hair loss improvement, hair root reinforcement, or inhibition of scalp inflammation.

The term "alleviation" as used herein may refer to any action that at least reduces a parameter related to the alleviation or treatment of a condition, for example, the degree of symptoms.

The term "hair growth promotion" as used herein may refer to all actions that increase hair growth in hair follicles, and may refer to all actions that increase the total amount of hair, such as, the proliferation of hair follicle cells, the activity of hair follicle cells, or the promoting the growth of hair follicle cells. "Hair growth promotion" is used interchangeably with "hair nurturing promotion" and refers to increasing the proportion of anagen-phase hair among all hair by promoting hair generation and growth. This includes not only promoting the generation of new hair, but also allowing existing hair to grow healthy.

The term "improvement of hair loss" as used herein may comprehensively mean a process of treating, mitigating, or alleviating a hair loss condition, or an effect thereof, and may refer to all actions of inhibiting the progress of hair loss, such as, for example, regeneration of hair follicle cells, restoration of hair follicle cell activity, inhibition of hair follicle cell apoptosis, and an increase in expression of factors related to hair root cell growth, such as VEGF. Therefore, the improvement of hair loss may encompass inhibition of hair loss, prevention of hair loss, treatment of hair loss, and the like.

The term "hair root reinforcement" as used herein refers to all actions that make the hair root part strong and healthy. For example, the hair root may be strengthened by proliferation of dermal papilla cells, promotion of nutrient delivery to dermal papilla cells, and an increase in expression of growth factors in dermal papilla cells. The composition according to an embodiment may be used to improve hair or scalp conditions by improving survival or proliferation of dermal papilla cells or by increasing expression of growth factors in dermal papilla cells.

The term "scalp inflammation" as used herein refers to inflammation occurring in the scalp. The scalp inflammation may include any one or more of seborrheic dermatitis, pruritus, dry eczema, erythema, urticaria, psoriasis, drug eruption, and scalp acne. The term "inhibition of scalp inflammation" as used herein refers to all actions of reducing or alleviating scalp inflammation or preventing or inhibiting the occurrence of scalp inflammation.

The "inflammation" may be associated with the enhancement of the repair system *in vivo* and biological damage, and it is known that regulating such processes is very complex. It is known that inflammation causes various inflammatory diseases and can cause hair loss if inflammation continues to occur in the scalp. The composition according to an embodiment may be used to improve hair or scalp conditions by alleviating scalp inflammation.

In an embodiment, the composition for improving hair or scalp conditions may enhance the viability of dermal papilla cells or increase the expression of vascular endothelial growth factor (VEGF) in dermal papilla cells.

According to an embodiment, it was confirmed that the composition for improving hair or scalp conditions has the effects of alleviating inflammation, promoting viability or cell activity of dermal papilla cells, promoting expression of hair growth-related factors, and promoting hair growth, and thus the composition may be used for improving hair or scalp conditions.

In an embodiment, the composition may be a composition for improving hair or scalp conditions and for external preparation for skin. The foregoing descriptions also apply equally to the composition for improving hair or scalp conditions and for external preparation for skin.

In the present specification, the term "external preparation for skin" is a concept encompassing the entire composition generally used for external use, and may be broadly applied as a cosmetic composition including various cosmetics such as basic cosmetics, makeup cosmetics, hair cosmetics, shaving cosmetics, and the like, or as various pharmaceuticals or quasi-drugs such as ointments.

The composition for external preparation for skin may be one further including, in addition to the active ingredient, components commonly incorporated into external preparation for skin according to the purpose of use and the nature of the composition for external preparation, specifically, a moisturizer, an ultraviolet absorber, vitamins, animal and plant extracts, a digestive agent, a whitening agent, a vasodilator, an astringent, a refreshing agent, a hormone agent, and the like.

The formulation of the composition for external preparation for skin may take an appropriate form depending on according to the purpose of use and the nature of the composition for external preparation, may specifically be an aqueous solution system, a solubilized system, an emulsion system, a milky lotion system, a gel system, a paste system, an ointment system, an aerosol system, a water-oil two-layer system, or a water-oil-powder three-layer system, and the formulation and form of the external preparation for skin of the present disclosure are not limited by the foregoing formulations.

In addition, the external preparation for skin may further include a base required to penetrate or transfer the active ingredient into the skin tissue.

In an embodiment, the composition may be a cosmetic composition having a formulation of shampoo, hair conditioner, hair lotion, hair essence, hair gel, hair pack, hair patch, or hair spray. The foregoing descriptions also apply equally to the cosmetic composition for improving hair or scalp conditions.

The cosmetic composition may be prepared in any formulation conventionally prepared in the art, and may be formulated into, for example, a solution, a suspension, an emulsion, a paste, a gel, a cream, a lotion, a powder, a soap, a surfactant-containing cleanser, an oil, a powder foundation, an emulsion foundation, a wax foundation, and a spray, but is not limited thereto. For example, The cosmetic composition may be prepared in the form of a softening lotion, a nourishing lotion, a nourishing cream, a massage cream, an essence, an eye cream, a cleansing cream, a cleansing foam, a cleansing water, a pack, a spray or a powder.

When the formulation of the cosmetic composition is a paste, cream, or gel, animal oil, vegetable oil, wax, paraffin, starch, tragacanth, cellulose derivatives, polyethylene glycol, silicone, bentonite, silica, talc, zinc oxide, or the like may be used as a carrier component. When the formulation of the cosmetic composition is a powder or a spray, lactose, talc, silica, aluminum hydroxide, calcium silicate, or polyamide powder may be used as a carrier component, and for example, when the formulation is a spray, a propellant such as chlorofluorohydrocarbon, propane/butane, or dimethyl ether may be additionally included.

When the formulation of the cosmetic composition is a solution or an emulsion, a solvent, a solubilizer, or an emulsifier is used as a carrier component, and the carrier component may include, for example, water, ethanol, isopropanol, ethyl carbonate, ethyl acetate, benzyl alcohol, benzyl benzoate, propylene glycol, 1,3-butylglycol oil, glycerol aliphatic ester, polyethylene glycol, or fatty acid ester of sorbitan.

When the formulation of the cosmetic composition is a suspension, a liquid diluent such as water, ethanol, or propylene glycol, a suspending agent such as ethoxylated isostearyl alcohol, polyoxyethylene sorbitol ester and polyoxyethylene sorbitan ester, and microcrystalline cellulose, aluminum metahydroxide, bentonite, agar, tragacanth, or the like may be used as a carrier component.

When the formulation of the cosmetic composition is a surfactant-containing cleanser, aliphatic alcohol sulfate, aliphatic alcohol ether sulfate, sulfosuccinic acid monoester, isethionate, imidazolinium derivatives, methyl taurate, sarcosinate, fatty acid amide ether sulfate, alkylamidobetaine, aliphatic alcohol, fatty acid glyceride, fatty acid diethanolamide, vegetable oil, lanolin derivatives, ethoxylated glycerol fatty acid ester, or the like may be used as a carrier component.

The cosmetic composition may include ingredients commonly used in cosmetic compositions, and may include, for example, conventional adjuvants such as antioxidants, stabilizers, solubilizers, vitamins, pigments, and fragrances

In an embodiment, the composition may have a formulation of shampoo, hair conditioner, hair lotion, hair essence, hair gel, hair pack, hair patch, or hair spray.

Another aspect provides a method of improving hair or scalp conditions, the method including applying a cosmetic composition including a chitosan derivative and a hydrolysate of a decellularized extracellular matrix to the skin of a subject. The foregoing descriptions also apply equally to the method for improving hair or scalp conditions. The terms "applying," "administering," and "applying" are used interchangeably and may refer to causing at least partial localization of the composition according to an embodiment to a desired site, or placing the composition according to an embodiment into a subject by a route of administration.

### Brief Description of Drawings

FIG. 1 shows results confirming the substitution of a methylcarboxyl group in a chitosan derivative in a solution including the chitosan derivative according to an embodiment by Fourier-transform infrared (FT-IR) spectroscopy.

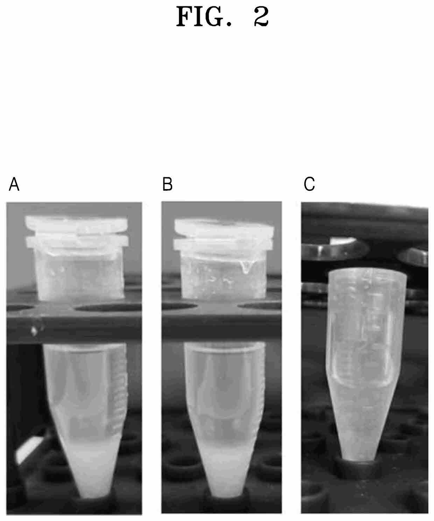

FIG. 2 shows a change in solubility of a chitosan derivative in a neutral solvent according to the degree of substitution of a methylcarboxyl group in a solution containing a chitosan derivative having a degree of deacetylation of 50% according to an embodiment, wherein A of FIG. 2 shows a chitosan derivative solution having a degree of substitution of a methylcarboxyl group of 0% as visually observed, B of FIG. 2 shows a chitosan derivative solution having a degree of substitution of a methylcarboxyl group of 50% as visually observed, and C of FIG. 2 shows a chitosan derivative solution having a degree of substitution of a methylcarboxyl group of 70% as visually observed.

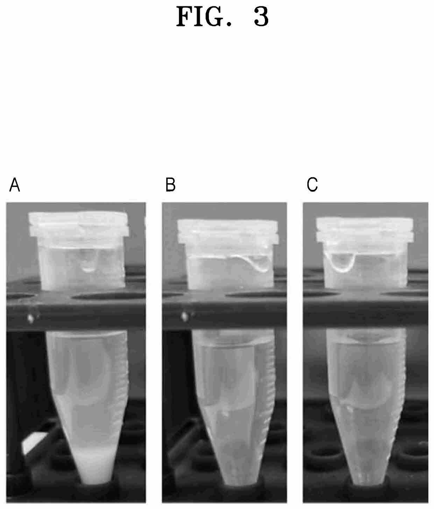

FIG. 3 shows a change in solubility of a chitosan derivative in a neutral solvent according to the degree of substitution of a methylcarboxyl group in a solution containing a chitosan derivative having a degree of deacetylation of 70% according to an embodiment, wherein A of FIG. 3 shows a chitosan derivative solution having a degree of substitution of a methylcarboxyl group of 0% as visually observed, B of FIG. 3 shows a chitosan derivative solution having a degree of substitution of a methylcarboxyl group of 50% as visually observed, and C of FIG. 3 shows a chitosan derivative solution having a degree of substitution of a methylcarboxyl group of 70% as visually observed.

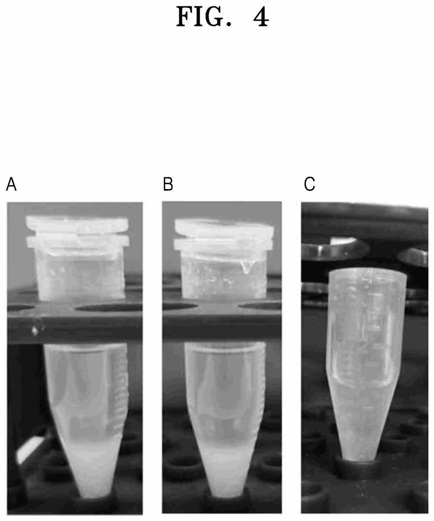

FIG. 4 shows a change in solubility of a chitosan derivative in a neutral solvent according to the degree of substitution of a methylcarboxyl group in a solution containing a chitosan derivative having a degree of deacetylation of 90% according to an embodiment, wherein A of FIG. 4 shows a chitosan derivative solution having a degree of substitution of a methylcarboxyl group of 0% as visually observed, B of FIG. 4 shows a chitosan derivative solution having a degree of substitution of a methylcarboxyl group of 50% as visually observed, and C of FIG. 4 shows a chitosan derivative solution having a degree of substitution of a methylcarboxyl group of 70% as visually observed.
FIG. 5 shows a change in solubility of a chitosan derivative according to a change in pH of a solution in a solution including a chitosan derivative having a degree of deacetylation of 90% and a degree of substitution of a methylcarboxyl group of 70% according to an embodiment, wherein A of FIG. 5 shows a chitosan derivative solution at pH 6.8 as visually observed, B of FIG. 5 shows a chitosan derivative solution at pH 6.4 as visually observed, and C of FIG. 5 shows a chitosan derivative solution at pH 6.1 as visually observed.
FIG. 6 shows the results of visually confirming the characteristics of the injectable liquid formulation of the *in vivo* ion-sensitive hydrogel composition according to an embodiment.
FIG. 7 shows results of confirming an inflammation-alleviating effect of treatment with a composition including a chitosan derivative according to an embodiment in peripheral blood mononuclear cells.
FIG. 8 shows the results of confirming and comparing the content of a residual active ingredient in a composition including a decellularized matrix hydrolysate according to an embodiment through a hydroxyproline assay and an elastin assay.
FIG. 9 shows the results of confirming the inflammation-alleviating effect of treatment with a solution containing a decellularized matrix according to an embodiment in peripheral blood mononuclear cells.
FIG. 10 is a view confirming the absorption of the decellularized matrix hydrolysate according to an embodiment into the dermal layer in porcine skin tissue.
FIG. 11 is a view confirming a cell proliferation effect of treatment with decellularized matrix hydrolysate according to an embodiment in dermal papilla cells.
FIG. 12 shows the results of measuring changes in expression level of a vascular endothelial growth factor following treatment with a decellularized matrix according to an embodiment in dermal papilla cells.
FIG. 13 shows the results of confirming a hair growth effect of treatment with a composition according to an embodiment in a mouse model in which skin is induced by Ultraviolet B (UVB) irradiation.

### Best Mode for Carrying out the Invention

Hereinafter, preferred examples are presented to help understanding of the present disclosure. However, the following examples are only provided to more easily understand the present disclosure, and the contents of the present disclosure are not limited by the following examples.

### [Example]

### Example 1. Evaluation of Dissolution Level of Chitosan Derivative Solution under Neutral Conditions

In conventional compositions containing chitosan, chitosan precipitates under neutral conditions, and the material stability is rapidly degraded upon mixing with other materials or components. Accordingly, this embodiment is to prepare a chitosan derivative solution having high solubility under neutral conditions.

### 1-1. Preparation of Chitosan Derivative Solution

solutions containing chitosan derivatives having different degrees of deacetylation (%) and degrees of substitution of methylcarboxyl groups (%), respectively, were prepared as follows. As shown in table 1 below, three groups were first classified based on the degree of deacetylation (50%, 70%, 90%) of the chitosan derivative, and the classified groups were reclassified into three experimental groups per each group based on the degree of substitution of the methylcarboxyl group in the chitosan derivative, and a total of nine experimental groups were set. In this regard, the degree of deacetylation (%) indicates a level in which an acetyl group is removed from chitin, which is a precursor of a chitosan derivative, and the degree of substitution (%) of a methylcarboxyl group indicates a level in which a hydroxy group and an amine group in the chitosan derivative are substituted with an -O-methylcarboxyl group or an -N-methylcarboxyl group. In addition, in the chitosan derivative solution, PBS (pH 7.4), which is a neutral solvent, was used as a solvent.

**[Table 1]**

| **Group** | **Degree of Deacetylation (%)** | **Degree of Substitution of Methylcarboxyl-Group (%)** | **Experimental Group** |
|---|---|---|---|
| G1 | 50 | 0 | G1-1 |
| | | 50 | G1-2 |
| | | 70 | G1-3 |
| G2 | 70 | 0 | G2-1 |
| | | 50 | G2-2 |
| | | 70 | G2-3 |
| G3 | 90 | 0 | G3-1 |
| | | 50 | G3-2 |
| | | 70 | G3-3 |

### (1) Preparation of G1-1 Experimental Group

10 g of chitin was reacted in 100 mL of a 50% NaOH solution at 100 °C for 30 minutes. Immediately after the reaction was completed, water was added to perform washing. The deacetylated chitosan was finally dried at 80 °C to finally obtain a chitosan powder having a degree of deacetylation of 50%.

### (2) Preparation of G1-2 Experimental Group

10 g of chitin was reacted in 100 mL of a 50% NaOH solution at 100 °C for 30 minutes. Immediately after the reaction was completed, water was added to perform washing. The deacetylated chitosan was finally obtained by drying at 80 °C. Thereafter, chitosan powder having a degree of deacetylation of 50% was added to 20% NaOH at 5 w/v%, and then monochloroacetic acid (1.0%) was added thereto and reacted at 40 °C. Thereafter, the chitosan derivative was neutralized with 10% acetic acid and then purified with methanol to finally obtain a chitosan derivative having a degree of deacetylation of 50% and 50% methylcarboxyl substitution degree.

### (3) Preparation of G1-3 Experimental Group

10 g of chitin was reacted in 100 mL of a 50% NaOH solution at 100 °C for 30 minutes. Immediately after the reaction was completed, water was added to perform washing. The deacetylated chitosan was finally obtained by drying at 80 °C. Thereafter, chitosan powder having a degree of deacetylation of 50% was added to 20% NaOH at 5 w/v%, and then monochloroacetic acid (1.5%) was added thereto and reacted at 40 °C. Thereafter, the chitosan derivative was neutralized with 10% acetic acid and then purified with methanol to finally obtain a chitosan derivative having a degree of deacetylation of 50% and a degree of substitution of a methylcarboxyl group of 70%.

### (4) Preparation of G2-1 Experimental Group

10 g of chitin was reacted in 100 mL of a 50% NaOH solution at 100 °C for 60 minutes. Immediately after the reaction was completed, water was added to perform washing. The deacetylated chitosan was finally dried at 80 °C to finally obtain a chitosan powder having a degree of deacetylation of 70%.

### (5) Preparation of G2-2 Experimental Group

10 g of chitin was reacted in 100 mL of a 50% NaOH solution at 100 °C for 60 minutes. Immediately after the reaction was completed, water was added to perform washing. The deacetylated chitosan was finally obtained by drying at 80 °C. Thereafter, chitosan powder having a degree of deacetylation of 70% was added to 20% NaOH at 5 w/v%, and then monochloroacetic acid (1.0%) was added thereto and reacted at 40 °C. Thereafter, the chitosan derivative was neutralized with 10% acetic acid and then purified with methanol to finally obtain a chitosan derivative having a degree of deacetylation of 70% and a degree of substitution of a methylcarboxyl group of 50%.

### (6) Preparation of G2-3 Experimental Group

10 g of chitin was reacted in 100 mL of a 50% NaOH solution at 100 °C for 60 minutes. Immediately after the reaction was completed, water was added to perform washing. The deacetylated chitosan was finally obtained by drying at 80 °C. Thereafter, chitosan powder having a degree of deacetylation of 70% was added to 20% NaOH at 5 w/v%, and then monochloroacetic acid (1.5%) was added thereto and reacted at 40 °C. Thereafter, the chitosan derivative was neutralized with 10% acetic acid and then purified with methanol to finally obtain a chitosan derivative having a degree of deacetylation of 70% and a degree of substitution of a methylcarboxyl group of 70%.

### (7) Preparation of G3-1 Experimental Group

10 g of chitin was reacted in 100 mL of a 50% NaOH solution at 100 °C for 150 minutes. Immediately after the reaction was completed, water was added to perform washing. The deacetylated chitosan was finally dried at 80 °C to finally obtain a chitosan powder having a degree of deacetylation of 90%.

### (8) Preparation of G3-2 Experimental Group

10 g of chitin was reacted in 100 mL of a 50% NaOH solution at 100 °C for 150 minutes. Immediately after the reaction was completed, water was added to perform washing. The deacetylated chitosan was finally obtained by drying at 80 °C. Thereafter, chitosan powder having a degree of deacetylation of 90% was added to 20% NaOH at 5 w/v%, and then monochloroacetic acid (1.0%) was added thereto and reacted at 40 °C. Thereafter, the chitosan derivative was neutralized with 10% acetic acid and then purified with methanol to finally obtain a chitosan derivative having a degree of deacetylation of 90% and a degree of substitution of a methylcarboxyl group of 50%.

### (9) Preparation of G3-3 Experimental Group

10 g of chitin was reacted in 100 mL of a 50% NaOH solution at 100 °C for 150 minutes. Immediately after the reaction was completed, water was added to perform washing. The deacetylated chitosan was finally obtained by drying at 80 °C. Thereafter, chitosan powder having a degree of deacetylation of 90% was added to 20% NaOH at 5 w/v%, and then monochloroacetic acid (1.5%) was added thereto and reacted at 40 °C. Thereafter, the chitosan derivative was neutralized with 10% acetic acid and then purified with methanol to finally obtain a chitosan derivative having a degree of deacetylation of 90% and a degree of substitution of a methylcarboxyl group of 70%.

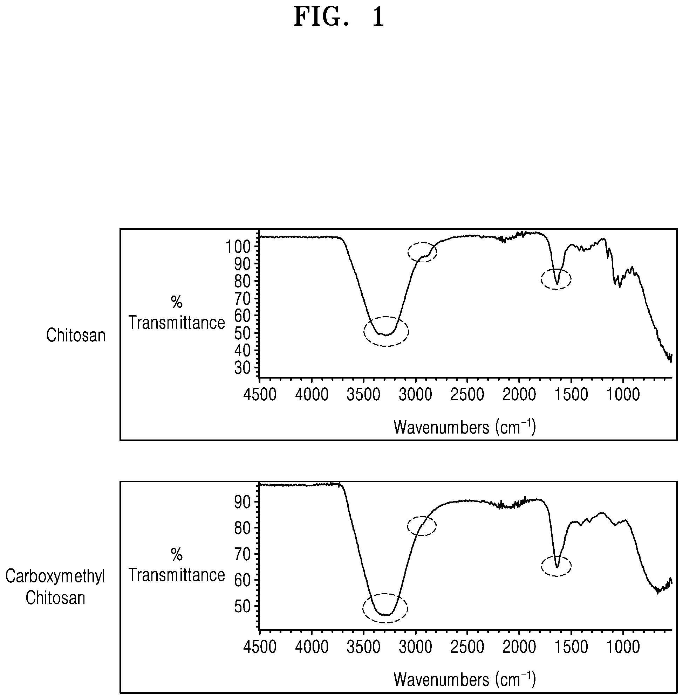

Thereafter, with respect to the chitosan derivatives having different degrees of deacetylation and degrees of substitution of a methylcarboxyl group obtained as described above, whether or not the chitosan derivative has been substituted with methylcarboxyl and the substitution degree thereof were confirmed by measuring the intensity of band through Fourier-transform infrared (FT-IR) spectroscopy (see FIG. 1) and measuring the content of free amine using Ninhydrin assay.

### 1-2. Evaluation of Dissolution Level of Chitosan Derivative in Neutral Solvent

In this example, by evaluating the dissolution level of the chitosan derivative solution of Example 1-1, the effect of the degree of deacetylation of the chitosan derivative and/or the degree of substitution of the methylcarboxyl group on the dissolution level of the chitosan derivative with respect to the neutral solvent was identified. To this end, the dissolution level of the chitosan derivative solution of Example 1-1 having different degrees of deacetylation and different degrees of substitution of a methylcarboxyl group at room temperature was evaluated as visually observed.

As a result, as shown in FIG. 2, it was observed that all of the G1 groups including the chitosan derivative having a degree of deacetylation of 50% produced precipitates with respect to the neutral solvent or were in a turbid state due to the low dissolution level of the chitosan derivative. On the other hand, as shown in FIGS. 3 and 4, as for the G2 group or the G3 group including the chitosan derivative having the degree of deacetylation of 70% or 90%, the solubility of the chitosan derivative in the neutral solvent was increased, and it was observed that the solution was in a transparent state. In particular, this effect was confirmed from the G2-2 group and the G3-2 group in which the degree of substitution of the methylcarboxyl group was 50% or more, and was more clearly observed as the degree of substitution of the methylcarboxyl group increased.

### Example 2. Evaluation of Dissolution Level of Chitosan Derivative Depending on Change in pH

In this example, by evaluating the change in the dissolution level of the chitosan derivative solution of Example 1 depending on the pH change, the difference from the conventional composition having the property of dissolving under acidic conditions was attempted to be verified. To this end, in the same manner as in Example 1, a solution containing a chitosan derivative having a degree of deacetylation of 90% and a degree of substitution of a methylcarboxyl group of 70% at a concentration of 2 w/v% was prepared. However, in the chitosan derivative solution, a 0.01N HCl aqueous solution, a 0.015N HCl aqueous solution, and a 0.02N HCl aqueous solution were used as solvents, respectively, and the pH level of the solution was adjusted to 6.8, 6.4, and 6.1, respectively. Thereafter, in the same manner as in Example 1-2, dissolution levels of chitosan derivative solutions having different pH values under room temperature conditions were evaluated as visually observed.

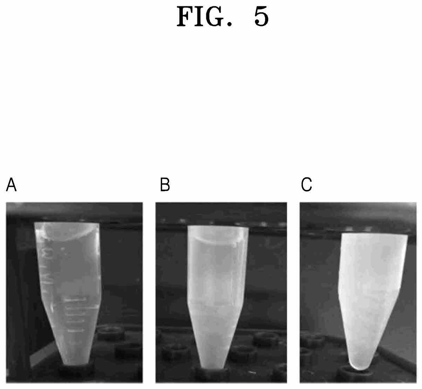

As a result, as shown in FIG. 5, under neutral conditions, the solution was observed to be in a transparent state due to the high degree of dissolution of the chitosan derivative, whereas under weakly acidic conditions of pH 6.5 or less, the solution was observed to be in a turbid state due to the formation of precipitates or the low degree of dissolution of the chitosan derivative.

Taken together, the experimental results indicate that, by adjusting the degree of deacetylation (70% or more) of the chitosan derivative and the degree of substitution of the methylcarboxyl group (50% or more), a chitosan derivative solution having high solubility and stability under neutral conditions, or a composition including the same, can be prepared, unlike conventional solutions including chitosan.

### Example 3. Evaluation of In Vivo Ion-Sensitivity Level According to Degree of the free amine groups

In order to be mixed with other water-soluble materials or components with high solubility and not to damage the effect of the active ingredient, the active ingredient should be present in an injectable liquid form under room temperature conditions, and gelation should proceed after administration into the body, that is, *in vivo,* to form an effective volume. To this end, in the present embodiment, the effect of the free amine group in the chitosan derivative on the *in vivo* ion-sensitivity behavior was examined.

### 3-1. Preparation of In Vivo Ion-Sensitive Hydrogel Composition

In this example, solutions containing chitosan derivatives having different degrees (%) of free amine groups were prepared as follows.

**[Table 2]**

| **Experimental Group** | **Degree of Deacetylation (%)** | **Degree of Substitution of Methylcarboxyl Group (%)** | **Free Amine Group (%)** |
|---|---|---|---|
| N1 | | 50 | 21 |
| N2 | 90 | 70 | 34 |
| N3 | | 0 | 90 |

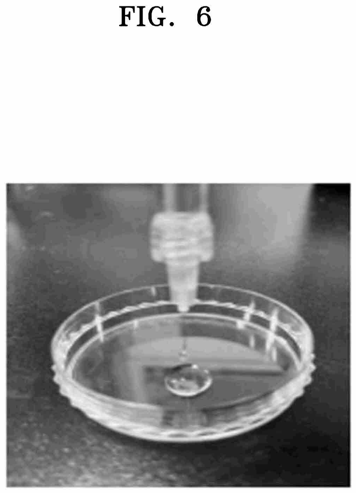

As shown in table 2, the degree of deacetylation (%) and the degree of substitution of the methylcarboxyl group (%) are as shown in table 1. The free amine group (%) represents the degree of free amine groups (-NH₂) among total amine groups in the chitosan derivative, and a disodium phosphate solution, which is a neutral solvent, was used as the solvent for the chitosan derivative solution. In the present example, the experimental groups (N1, N2, N3) were prepared by performing N, O-random carboxymethyl substitution, and the degree of the free amine groups was adjusted by adjusting the degree of substitution of a methylcarboxyl group in the same manner as in Example 1-1. Thereafter, the 2 w/v% chitosan derivative solution and 2 v/v % sodium phosphate dibasic (disodium hydrogen phosphate, Na₂HPO₄) were mixed to prepare a liquid chitosan derivative composition as shown in FIG. 6.

Taken together, the experimental results indicate that, by adjusting the degree of deacetylation (70% or more) of the chitosan derivative and the degree of substitution of the methylcarboxyl group (50% or more), a chitosan derivative solution having high solubility and stability under neutral conditions, or a composition including the same, can be prepared, unlike conventional solutions including chitosan.

### Example 4. Confirmation of Inflammation- Alleviating Effect of Composition Containing Chitosan Derivative

In this example, in order to confirm the inflammation-alleviating effect of the composition containing the chitosan derivative, a solution containing the chitosan derivative was prepared and a change in the expression level of TNF-α, which is an inflammation-related factor, according to the treatment therewith was measured.

### 4-1. Preparation of Composition Including Chitosan Derivative

Chitosan derivative powder having a degree of deacetylation of 90%, a degree of substitution of the methylcarboxyl group of 80%, and a free amine group content of 27.6% was added in an amount of 0.2 w/v% to a 0.05N HCl solution, and reacted at 60 °C for 4 hours. Thereafter, the solution was neutralized to a pH of 6.5 to 7.0 with a 0.5 M sodium phosphate solution, thereby preparing a composition including a chitosan derivative.

### 4-2. Confirmation of Inflammation-Alleviating Effect of Composition Containing Chitosan Derivative

In order to confirm the inflammation-alleviating effect of a composition including a chitosan derivative according to an embodiment, peripheral blood mononuclear cells (PBMCs) were stimulated with LPS (lipopolysaccharide) at a concentration of 10 µg/mL to induce an inflammatory response. PBMCs were seeded in a 24-well plate at a density of 4 × 10⁵ cells/well, and a mixture prepared by mixing the composition of Example 4-1 with a cell culture medium (LGM-3 medium (10% FBS, DNase 20 units/mL)) at a ratio of 1:1 was added to each well in an amount of 2 mL, followed by incubation at 37 °C for 48 hours. The culture medium was collected, and changes in the expression level of tumor necrosis factor-alpha (TNF-α) in the cells were measured through western blot analysis. As a comparative group, a group treated by mixing a hyaluronic acid solution (2 w/v%) with a cell culture solution at a ratio of 1:1 (comparative group 1) and a group treated with dexamethasone at a concentration of 0.4 µg/mL (comparative group 2) were used.

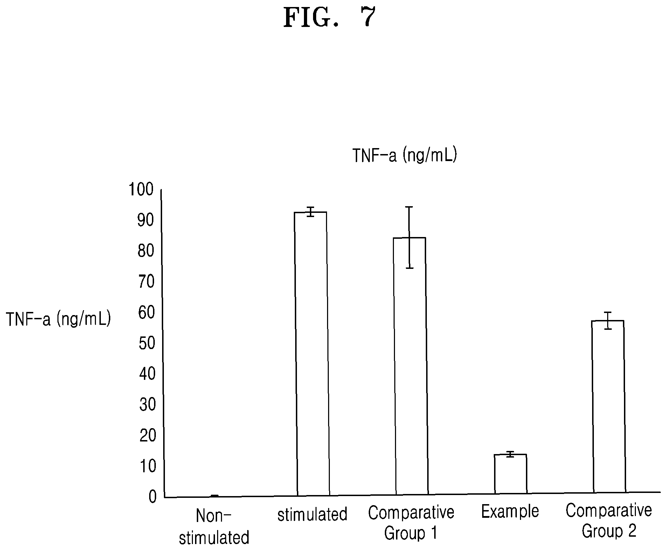

As a result, as shown in FIG. 7, it was confirmed that the degree of TNF-α increased by stimulation was significantly reduced by treatment with the composition containing the chitosan derivative according to an embodiment. In particular, the inflammation-alleviating effect was found to be significantly superior to that of the hyaluronic acid-treated group or the dexamethasone-treated group. Through these results, it was found that the composition containing the chitosan derivative according to an embodiment had an excellent inflammation-alleviating effect.

### Example 5. Confirmation of Hair or Scalp Improvement Effect of Decellularized Extracellular Matrix Hydrolysate

In this example, to evaluate the hair and scalp improvement effects of a composition including a hydrolysate of a decellularized extracellular matrix (hereinafter, 'decellularized matrix') according to an embodiment, changes in the expression levels of collagen and elastin, which are factors associated with hair and scalp improvement, were measured according to the presence or absence of hydrolysis and the specific hydrolysis method used.

### 5-1. Preparation of Decellularized Extracellular Matrix Composition

Porcine skin tissue was prepared and keratin was removed with a knife, and pretreated to remove fat by stirring with 70% ethanol at room temperature for 4 hours. Thereafter, 20 g of the skin tissue and 100 ml of 100% ethanol were put into a supercritical fluid equipment (Ilshin Autoclave, Korea) reactor, and the temperature was set to 35 °C. Thereafter, carbon dioxide was injected to adjust the pressure under the condition of 300 bar, and the reaction was performed for 6 hours under the temperature and pressure conditions. Thereafter, the tissue was washed with an ethanol solution for 2 hours, followed by washing with phosphate-buffered saline (PBS) and distilled water for 4 hours.

The washed tissue was freeze-dried and powdered using a freeze-miller to obtain a decellularized extracellular matrix (hereinafter, decellularized matrix) dry powder.

### 5-2. Preparation of Decellularized Extracellular Matrix Hydrolysate under Basic Conditions and Confirmation of Effect of Improving Hair or Scalp Thereof

The decellularized matrix dry powder prepared according to Example 5-1 was mixed in a 0.2 N NaOH solution at a concentration of 2.0 w/v% and reacted at 80 °C for 16 hours. Subsequently, the mixture was neutralized to a final pH of 7.4 using an HCl solution (final concentration: 20 mg/ml).

In comparison, the decellularized matrix dry powder prepared in Example 5-1 was treated with an acid solution (Comparative Example 1) or a solution containing an acid and an enzyme (Comparative Example 2). Thereafter, the contents of remaining factors associated with hair and scalp improvement were compared among the groups.

### Comparative Example 1. Preparation of Decellularized matrix hydrolysate Using Acid

The dry powder of the decellularized matrix prepared according to Example 5-1 was mixed in a 0.1N HCl solution at 2.0 w/v%. Thereafter, the temperature of the solution was reacted at 80 °C for 16 hours, and then the precipitate was removed. Thereafter, the pH was neutralized to be 7.4 using a NaOH solution (final concentration: 20 mg/ml).

### Comparative Example 2. Preparation of Decellularized Matrix Hydrolysate Using Acid and Enzyme

10 wt% of pepsin based on the decellularized matrix was mixed in the 0.1N HCl solution, and then the dry powder of the decellularized matrix prepared according to Example 5-1 was mixed in an amount of 2.0 w/v%. Thereafter, the solution was reacted at 4 °C for 24 hours, and then the solution was filtered using a 100 kDa filter. Thereafter, the pH was neutralized to be 7.4 using a NaOH solution (final concentration: 20 mg/ml).

### 5-3. Measurement of Content of Factors Related to Improvement of Scalp or Hair Condition

In order to confirm the contents of collagen and elastin, which are factors related to improvement of scalp or hair condition, a hydroxyproline assay and an elastin assay were performed as follows.

### 5-3-1. Hydroxyproline Assay for Measuring Residual Collagen Content

12N HCl was mixed in the decellularized matrix hydrolysate according to Comparative Example 1, Comparative Example 2, or Example 5-2 at a ratio of 1:1 (v/v), and then hydrolyzed at 100 °C for 3 hours. The hydroxyproline standard solution and the hydrolyzed sample were dispensed into a 96-well plate to completely evaporate the solvent. Thereafter, the chloramine-T reagent was added to the well where the sample was dispensed, and then reacted at room temperature for 5 minutes. After completion of the reaction, DMAB reagent was added thereto, and the reaction was performed at 60 °C for 90 minutes. After the reaction was completed, absorbance was measured at a wavelength of 560 nm. The hydroxyproline standard curve was constructed to calculate the hydroxyproline content of the sample, and then the collagen content was converted and quantified.

### 5-3-2. Elastin Assay for Measuring Residual Elastin Content

Solutions containing the decellularized matrix according to Comparative Example 1, Comparative Example 2, or Example 5-2 were each mixed with oxalic acid and then hydrolyzed at 100 °C. A supernatant containing elastin was obtained using a centrifuge. An elastin precipitation reagent was added to the supernatant sample and the elastin standard solution thus obtained, and cultured at 4 °C, and then the elastin precipitate was obtained using a centrifuge. Thereafter, a staining reagent was added thereto, and after treatment at 4 °C, centrifugation was performed to obtain stained elastin pellets. The sample containing the pellets was treated with a staining dissociation reagent, and then absorbance was measured at a wavelength of 513 nm. Elastin content was calculated using the elastin standard curve.

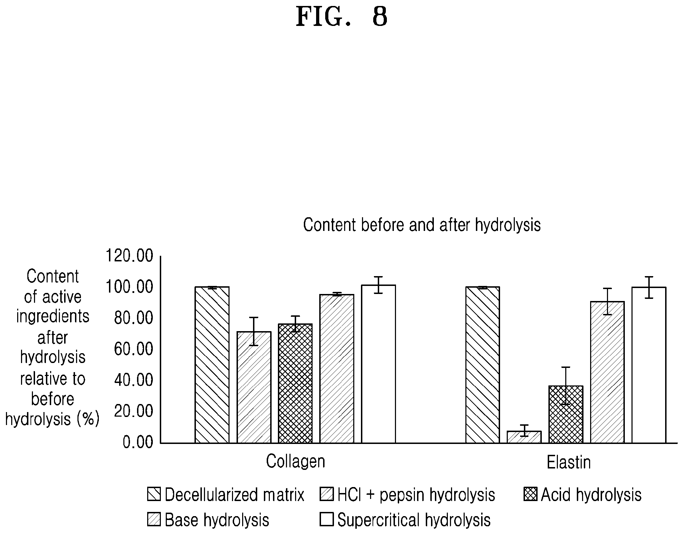

As a result, as shown in FIG. 8, it was confirmed that when hydrolysis was performed by treatment with an acid or an acid and an enzyme, the contents of collagen and elastin in the decellularized matrix hydrolysate were significantly reduced accordingly. In contrast, it was confirmed that when hydrolysis was performed by base treatment, the contents of collagen and elastin in the decellularized matrix hydrolysate were maintained before and after the hydrolysis. Through these results, it was found that the decellularized matrix hydrolysate subjected to the hydrolysis process under basic conditions according to an embodiment had an excellent hair or scalp improvement effect.

### 5-4. Confirmation of Inflammation-alleviating effect

In this example, to investigate the inflammation-alleviating effect of the decellularized matrix hydrolysate according to an embodiment, changes in TNF-α expression levels were measured in cells where an inflammatory response had been induced.

Specifically, for PBMCs that induced an inflammatory response in the same manner as in Example 4-2, the decellularized material solutions according to Example 5-2 and Comparative Example 2 were mixed and treated with a cell culture solution at a ratio of 1:1 (1 w/v%), and then the change in TNF-α expression level was measured through Western blot analysis. As a negative control group, an untreated group was used, and as a comparative group, a group treated with Comparative Example 1 (Comparative Group 1) and a group treated with dexamethasone at a concentration of 0.4 µg/mL (Comparative Group 2) were used.

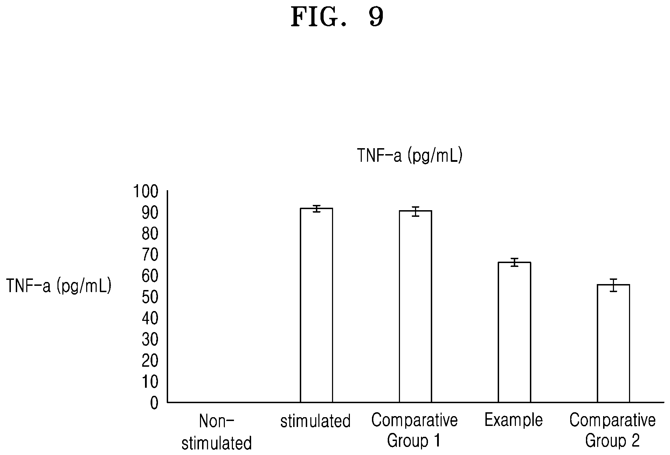

As a result, as illustrated in FIG. 9, a decrease in the TNF-α expression level was confirmed after treatment with the decellularized extracellular matrix hydrolysate. Accordingly, it was confirmed that the composition according to an embodiment can exhibit excellent hair or scalp condition improvement effects due to an excellent inflammation-alleviating effect.

### 5-5. Confirmation of Absorption to Dermal Layer

In this example, in order to confirm the degree of absorption of the decellularized matrix hydrolysate according to an embodiment into the dermal layer, an experiment was performed as follows.

Specifically, fluorescence (DyLight 800 NHS Ester) was added to the decellularized matrix hydrolysates according to Example 5-2 and Comparative Example 2, and the fluorescently tagged decellularized matrix solutions were applied to porcine skin at a concentration of 100 µl/cm². Thereafter, the tissue was cross-sectioned and observed under a fluorescence microscope.

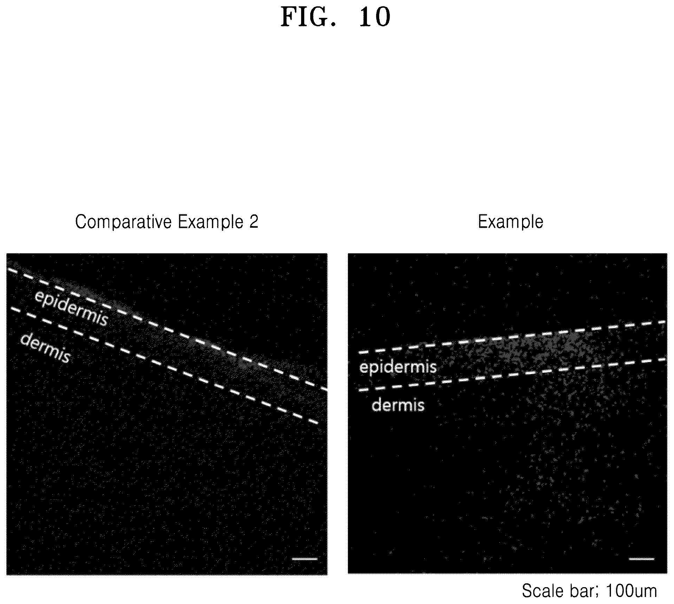

As a result, as shown in FIG. 10, it was confirmed that the decellularized matrix according to an embodiment was absorbed to the dermal layer. In particular, the hydrolyzed decellularized matrix was found to be absorbed into the dermal layer compared with the matrix before hydrolysis. As a result, it was confirmed that the composition according to an embodiment may exhibit an excellent hair or scalp improvement effect by delivering a bioactive material (e.g., collagen or elastin) *in vivo* with excellent efficiency.

### 5-6. Confirmation of Hair Root Cell Proliferation and Enhancement Effect

In this example, in order to confirm the effect of improving or enhancing the scalp or hair condition of the decellularized matrix hydrolysate according to an embodiment, the cell proliferation level for hair root cells and the expression level of related growth factors in hair root cells according to the decellularized material hydrolysate treatment were measured.

Specifically, the decellularized material hydrolysate of Example 5-2 was diluted in DPBS at concentrations of 2 mg/mL, 4 mg/mL, and 10 mg/mL, and then mixed with a cell culture solution at a concentration ratio of 1:1 (0.1 w/v%, 0.2 w/v%, and 0.5 w/v%, respectively). Human dermal papilla cells were inoculated into 24-well at 1X104 cells/well, and then the mixed solution was treated at a concentration of 2 mL/well, and then cultured at 37 °C, and changes in cell viability and expression levels of vascular endothelial growth factor (VEGF) were measured over time. As a negative control, a group treated with DPBS was used, and as a positive control, a group treated with a cell culture medium was used. The cell viability was measured using a CCK-8 assay kit, and specifically, the cells were treated with a solution in which the cell culture medium and the CCK-8 reagent were mixed at a ratio of 10:1, cultured in an incubator at 37 °C for 4 hours, and then absorbance was measured at 450 nm. VEGF expression levels were measured using VEGF elisa kit (R&D systems).

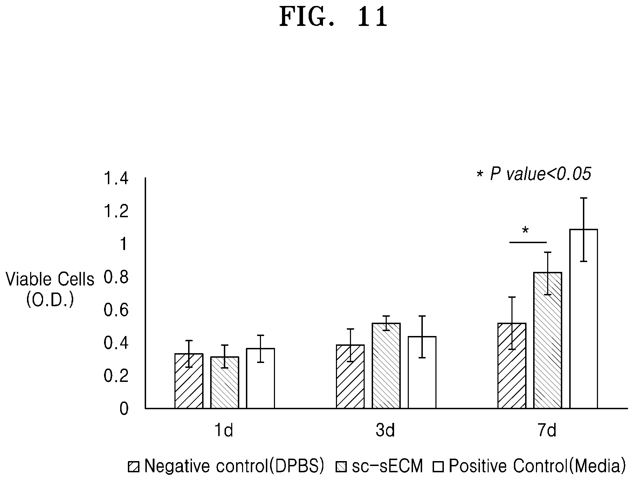

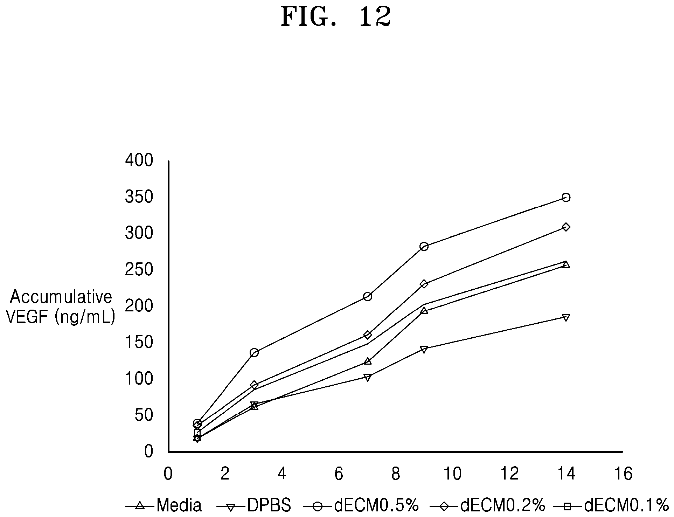

As a result, as shown in FIG. 11, the viability of hair root cells was found to increase after 3 days following treatment with the hydrolyzed decellularized matrix according to an embodiment, and in particular, the viability of the hair root cells significantly increased after 7 days. In addition, as shown in FIG. 12, it was observed that the expression level of VEGF was significantly increased over time by treating the hair root cells with the hydrolyzed decellularized matrix according to an embodiment. In addition, it was found that as the content of the decellularized matrix increased, the increase in VEGF expression was greater.

Through these results, it was confirmed that the composition including the decellularized material hydrolysate according to an embodiment has an excellent scalp or hair improvement or enhancement effect.

### Example 6. Preparation of Composition Containing Chitosan Derivative and Extracellular Matrix and Confirmation of Hair Growth Effect Thereof

In this example, in order to confirm the hair growth effect of the composition including the chitosan derivative and the decellularized matrix hydrolysate, an experiment was performed as follows.

Specifically, chitosan derivative powder having a degree of deacetylation of 90%, a degree of substitution of the methylcarboxyl group of 80%, and a free amine group content of 27.6% was added in an amount of 0.2 w/v% to a 0.001N HCl solution, and reacted at 60 °C for 4 hours. Thereafter, the mixture was neutralized to a pH of 7.0 to 7.5 with a 0.5 M sodium phosphate solution, thereby preparing a composition including a chitosan derivative (Example 6-1).

In addition, the chitosan derivative powder was added to a 0.001 N HCl solution at 0.4 w/v%, followed by reaction at 60 °C for 4 hours. The resulting solution was then neutralized to a pH of 7.0 to 7.5 with a 0.5 M sodium phosphate solution, after which the decellularized matrix hydrolysate of Example 5-2 was mixed therewith to prepare a mixture having concentrations of 0.2 w/v% carboxymethyl chitosan and 0.5 w/v% decellularized matrix hydrolysate (Example 6-2).

Thereafter, in a mouse model in which scalp inflammation and hair loss were induced by irradiating the dorsal skin of mice with UVB twice at 200 mJ/cm² on an area of 6 cm², 300 µL of the compositions of Examples 6-1 and 6-2 were applied to the lesion site of the mice at 50 µL/cm² once daily for 14 days, and the change in hair growth area was then measured. As a negative control group, a group treated with saline was used, and as a comparative group, a group treated with minoxidil at a concentration of 50 µl/cm2 was used.

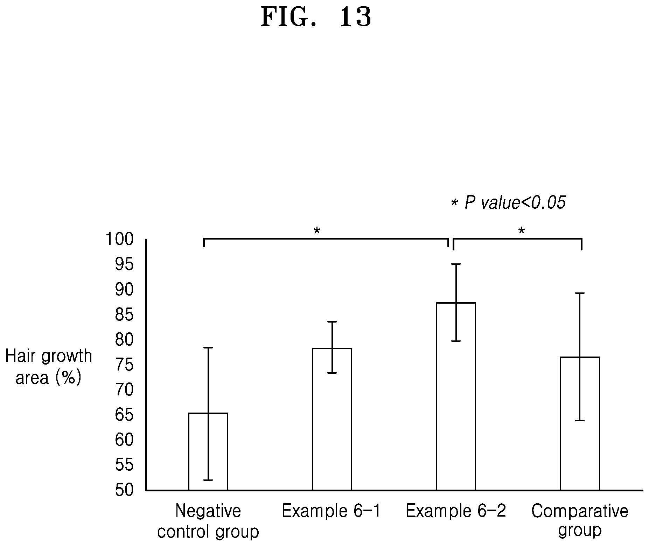

As a result, as shown in FIG. 13, it was confirmed that the composition including the chitosan derivative and the decellularized material according to an embodiment may significantly increase the hair growth area of the mouse. In particular, it was confirmed that the hair growth-promoting effect thereof was remarkably superior to that of the comparative group.

The above description of the present disclosure is provided for illustrative purposes, and those skilled in the art will understand that the present disclosure may be easily modified into other specific forms without departing from the technical spirit or essential features of the present disclosure. Therefore, it should be understood that the embodiments described above are exemplary and not restrictive in all aspects.

## Claims

1. A pharmaceutical composition for preventing or treating hair loss, comprising: a chitosan derivative satisfying the following conditions; and
a hydrolysate of a decellularized extracellular matrix:
(a) a degree of deacetylation is 70% or more;
(b) 50% or more of hydroxyl groups and amine groups in the chitosan derivative are substituted with an O-alkylcarboxyl group or an N-alkylcarboxyl group; and
(c) 22% or more of total amine groups in the chitosan derivative are free amine groups (-NH₂).

2. The composition of claim 1, wherein the chitosan derivative has a degree of deacetylation of 70% to 95%.

3. The composition of claim 1, wherein 50% to 90% of hydroxyl groups and amine groups in the chitosan derivative are substituted with an O-methylcarboxyl group or an N-methylcarboxyl group.

4. The composition of claim 1, wherein the chitosan derivative has 22% to 90% free amine groups based on the total amine groups in the chitosan derivative.

5. The composition of claim 1, wherein the chitosan derivative has an average molecular weight of 50,000 to 3,000,000 Da.

6. The composition of claim 1, wherein the composition has a pH of 6.5 to 8.0.

7. The composition of claim 1, wherein the free amine group (-NH₂ in the chitosan derivative is present in the form of -NH₃+.

8. The composition of claim 1, wherein the decellularized extracellular matrix is prepared by a decellularization method comprising injecting supercritical carbon dioxide into a reactor containing a biological tissue and ethanol such that a pressure in the reactor is 70 to 400 bar, and the injecting the supercritical carbon dioxide into the reactor containing the biological tissue and ethanol is performed at 31 to 60 °C.

9. The composition of claim 1, wherein the hydrolysate of the decellularized extracellular matrix is obtained by subjecting the decellularized extracellular matrix to treatment with a base solution, treatment with supercritical carbon dioxide, or a sequential combination of said treatments.

10. The composition of claim 1, wherein the composition increases viability of human dermal papilla cells or expression of vascular endothelial growth factor (VEGF) in human dermal papilla cells.

11. A composition for improving hair or scalp conditions, the composition comprising: a chitosan derivative satisfying the following conditions; and
a hydrolysate of a decellularized extracellular matrix:
(a) a degree of deacetylation is 70% or more;
(b) 50% or more of hydroxyl groups and amine groups in the chitosan derivative are substituted with an O-alkylcarboxyl group or an N-alkylcarboxyl group; and
(c) 22% or more of total amine groups in the chitosan derivative are free amine groups (-NH₂).

12. The composition of claim 11, wherein the improvement in hair or scalp condition is at least one of hair growth promotion, hair loss prevention, hair root strengthening, or scalp inflammation suppression.

13. The composition of claim 11, wherein the composition is a composition for external preparation for skin for improving hair or scalp conditions.

14. The composition of claim 11, wherein the composition is a cosmetic composition having a formulation of shampoo, hair conditioner, hair lotion, hair essence, hair gel, hair pack, hair patch, or hair spray.
